# EUROPÄISCHE PATENTSCHRIFT

(11) **EP 1 392 285 B1**
(45) Veröffentlichungstag und Bekanntmachung des Hinweises auf die Patenterteilung: **09.02.2005**
(21) Anmeldenummer: 02747189.5
(22) Anmeldetag: 24.05.2002
(51) Int. Cl.: A61K 31/385, A61K 31/401, A61K 31/403, A61K 31/137, A61P 13/12

(54) **ARZNEIMITTEL ENTHALTEND EINEN EFFEKTOR DES GLUTATHIONMETABOLISMUS ZUSAMMEN MIT ALPHA-LIPONSÄURE IM RAHMEN DER NIERENERSATZTHERAPIE**
MEDICAMENT CONTAINING AN EFFECTOR OF THE GLUTATHIONE METABOLISM TOGETHER WITH ALPHA-LIPOIC ACID FOR USE IN KIDNEY REPLACEMENT THERAPY
MEDICAMENT RENFERMANT UN EFFECTEUR DE METABOLISME DU GLUTATHION CONJOINTEMENT AVEC DE L'ACIDE ALPHA-LIPOIQUE, UTILISE EN THERAPIE DE REMPLACEMENT DU REIN

(30) Priorität: 28.05.2001 DE 10125883
(43) Veröffentlichungstag der Anmeldung: 03.03.2004
(73) Patentinhaber: Serumwerk Bernburg AG, 06406 Bernburg (DE)
(72) Erfinder: TÄGER, Michael, 39326 Heinrichsberg (DE); ANSORGE, Siegfried, 39291 Hohenwarthe (DE); FRIES, Gerhard, 39171 Osterweddingen (DE); KOEGST, Dieter, 39171 Osterweddingen (DE)
(74) Vertreter: Müller, Wolfram Hubertus, Dr. Dipl.-Phys.
(86) Internationale Anmeldenummer: PCT/DE2002/001991
(87) Internationale Veröffentlichungsnummer: WO 2002/096414

(56) Entgegenhaltungen:
- EP-A- 0 427 247
- DE-A- 3 804 858
- DE-A- 4 420 102
- HAN D ET AL: "A-LIPOIC ACID INCREASE INTRACELLULAR GLUTATHIONE IN A HUMAN T-LYMPHOCYTE JURKAT CELL LINE" BIOCHEMICAL AND BIOPHYSICAL RESEARCH COMMUNICATIONS, ACADEMIC PRESS INC. ORLANDO, FL, US, Bd. 207, Nr. 1, 1995, Seiten 258-264, XP000887357 ISSN: 0006-291X

## Beschreibung

Die Erfindung betrifft die Verwendung der Kombination von α-Liponsäure und Effektoren des Glutathionmetabolismus zur Behandlung von Störungen des zellulären Thiolstatus und damit einhergehenden Erkrankungen im Rahmen der Nierenersätztlierapie.

Die Feinregulation des Thiol-Disulfid-Status stellt eine der wichtigsten Grundvoraussetzungen biologischer Stoffwechselleistungen dar. Das zentrale Regulationselement innerhalb dieses Systems ist das Tripeptid Glutathion, welches intrazellulär in reduzierter Form relativ hohe Konzentrationen (bis zu 10 mM) erreicht. Neben dem Glutathion sind Thiol-Gruppen, tragende Proteine intrazellulär und insbesondere in zellmembrangebundener Form weitere bedeutende Bausteine des Thiol-Disulfidstatus jeder Zelle.

Der durch verschiedene Enzymklassen regulierte Metabolismus der Disulfidspaltung und Thiolgruppenbildung ist durch die Vielfalt seiner biologischen Funktionen u. a. bei zellulären Wachstums- und Differenzierungsprozessen einschließlich des programmierten Zelltodes sowie Zellschutz- und Entgiftungsmechanismen in seiner Intaktheit unabdingbar für jede normale Zellfunktion. Störungen in diesem System und Veränderungen der Konzentration der Thiole führen zu schwerwiegenden zellulären Funktionsstörungen, die nur im Einzelfall lokal begrenzt bleiben, in der Regel jedoch den gesamtem Organismus beeinträchtigen.

In einer Vielzahl von Untersuchungen konnte die Beteiligung eines gestörten Thiol-Disulfid-Status bei akuten und chronischen Erkrankungen nachgewiesen werden.

So wurden beispielsweise in bestimmten Nervenzellen bei neurodegenerativen Erkrankungen wie der Parkinsonschen Krankheit deutliche Veränderungen des Thiolstoffwechsels nachgewiesen (Brain Res Rev 1997;25:335-358). Es gibt deutliche Hinweise darauf, daß es in Folge dieser Stoffwechselstörung zu einem vermehrten Untergang der für die Symptomatik der Erkrankung maßgeblich verantwortlichen Nervenzellen in funktionell beeinträchtigten Hirnarealen, den Basalganglien, kommt (Ann Neurol 1994;36:348-355).

Verringerte Glutathionspiegel bzw. ein verringerter intrazellulärer Glutathiongehalt wurde weiterhin im Rahmen von Gefäßerkrankungen und deren Folgezuständen - Arteriosklerose und Herzinfarkt - in den, die Gefäßinnenwand auskleidenden Endothelzellen gefunden (Med Sci Res 1998;26:105-106).

Lungenerkrankungen, welche mit einem Umbau des Lungengewebes einhergehen, sind regelmäßig mit einem Glutathiondefizit im Gewebe verbunden. Bei einer solchen Lungenfibrose verläuft der Schweregrad der Erkrankung parallel zum Thiolverlust (Clin Chim Acta 1997;265:113-119). Schwere entzündliche Lungenerkrankungen, untersucht am Beispiel des akuten Atemnotsyndromes des Erwachsenen, werden von einer Dysregulation des Thiolstoffwechsels der beteiligten Entzündungszellen (Granulozyten) begleitet (Chest 1996;109: 163-166).

Immunkompetente Abwehrzellen des Bronchialsystems (Alveolarmakrophagen) von Rauchern und Patienten mit chronisch-obstruktiven Atemwegserkrankungen weisen nach eigenen Untersuchungen ein schweres zelluläres Thioldefizit auf. Der Grad der Störung des zellulären Thiolstatus korreliert dabei direkt mit Einschränkungen der Lungenfunktion (Free Radic Biol Med 2000; 29:1160-1165).

In den letzten Jahren wurden darüber hinaus vermehrt Hinweise auf einen geschädigten Thiolstoffwechsel bei chronischen Nierenerkrankungen (Ren Fail 1998;20:117-124), Anämien (Br J Haematol 1995;91:811-819), unreifen Neugeborenen (Pediatr Pulmonol 1995;20: 160-166), lärmbedingtem Hörverlust (Brain Res 1998;784:82-90), entzündlichen Darmerkrankungen (Gut 1998;42:485-492) sowie bei Diabetes mellitus (Metabolism: Clinical and Experimental 1998; 47(8):993-997) gefunden.

Umfangreiche Untersuchungen zur Bedeutung des Glutathionstoffwechsels bei Virusinfektionen wiesen sowohl eine, auf einer geschädigten zellulären Abwehr basierende, schlechtere Prognose thioldefizienter Zellen als auch eine antivirale, die Virusvermehrung hemmende Funktion des Glutathions nach (Proc Natl Acad Sci USA 1997;94:1967-1972).

Das zelluläre Immunsystem des Menschen, bestehend aus den weißen Blutzellen Granulozyten, Lymphozyten und Monozyten stellt ein auf eine Störung im Thiolstoffwechsel besonders empfindlich reagierendes System dar.

Minimale Änderungen, insbesondere Verluste zellulären Glutathions können ein kaskadenartiges Programm zur Selbstzerstörung der Zelle, den programmierten Zelltod (Apoptose) auslösen (FASEB J 1998;12:479-486). Der Thiol-Disulfidstoffwechsel wirkt hier als ein zentrales Stellglied eines intakten Immunsystems, ohne welches der Organismus nicht lebensfähig wäre.

Eigene Untersuchungen zeigten, daß insbesondere unter den Bedingungen einer hochgradig eingeschränkten Nierenfunktion und dadurch erforderlicher Nierenersatztherapie in Form der Hämo- bzw. Peritonealdialyse der zelluläre Thiol-Disulfidstoffwechsel schwer gestört ist. Diese Störung hat u.a. einen weitgehendenen Verlust normaler Zellfunktionen, wie der Phagozytosefähigkeit von Peritonealmakrophagen oder der Aktivierbarkeit von Lymphozyten zur Folge. Regelmäßig werden bei diesen Patienten neben dem bestehenden lokalen Immundefizit, welches durch häufige Infektionen der Bauchhöhle gekennzeichnet ist, auch eine deutlich verringerte immunologische Abwehr mit gesteigerter allgemeiner Infektanfälligkeit gefunden. Beschrieben sind hier insbesondere Funktionsstörungen und eine verringerte Aktivierbarkeit der Lymphozyten und Makrophagen sowie Imbalancen der immunregulatorischen Zytokine (Immunobiol 1999;200:62-76).

Die Korrektur eines gestörten Thiolstoffwechsels erlangt somit grundlegende Bedeutung als Basistherapie bei der Behandlung einer Vielzahl von Erkrankungen unterschiedlicher Genese, insbesondere jedoch unter den Bedingungen einer notwendigen Nierenersatztherapie.

α-Liponsäure wird bislang mit relativem Erfolg zur Behandlung von neurotoxisch bedingten Mißempfindungen im Rahmen der diabetischen Polyneuropathie als neuroprotektive Substanz eingesetzt (Diabetologica 1995; 38:1425-1433, Diabetes Res Clin Pract 1995;29:19-26, Diab Care 1999;22:1296-1301, Drug Metab Rev 1997; 29:1025-1054, DE 43 43 592 C2). Aus der DE 44 47 599 C2 und der EP 0 530 446 B1 ist darüber hinaus die Verwendung von α-Liponsäure bei weiteren neuronalen Störungen einschließlich Tinnitus und Hörsturz bekannt.

Der zytoprotektive Wirkmechanismus hier beruht neben der Beeinflussung der zuckerabhängigen Proteinmodifkation (Proteinglykosylierung) sowie einer Verringerung der neurotoxischen Ketonkörpergenese letztendlich auf der antioxidativen Funktion der α-Liponsäure und deren Metabolite (Free Radic Biol Med 1995;19:227-250).

Diese Zellschutzfunktion wurde besonders unter dem Aspekt der Verhinderung des oxidativen Umbaus von essentiellen ungesättigten Fettsäuren untersucht. Eine solche Hemmung der Lipidperoxidation stellt neben der Anwendung der α-Liponsäure als Neuroprotektivum die Basis für eine Applikation als Leberschutzmedikament bei verschiedenen Intoxikationen und Lebererkrankungen dar (Biochemistry 1998;37:1357-1364).

Darüber hinaus konnte gezeigt werden, daß α-Liponsäure die Vermehrung des HI-Virus auf unterschiedlichen Entwicklungsstufen hemmt und somit einer Progression der AIDS-Erkrankung entgegenwirken könnte. Die Ergebnisse dieser Laborversuche konnten allerdings nur eingeschränkt auf klinische Studien übertragen werden (FEBS-Lett 1996;394:9-13). Ähnliches gilt für den Nachweis einer entzündungshemmenden Funktion der Substanz für die insulinproduzierenden Inselzellen der Bauchspeicheldrüse (Agents Actions 1993;38:60-65).

In der EP 0 812 590 A2 sowie der EP 0 427 247 B1 wird die Verwendung von α-Liponsäure als Zytoprotektivum, Antischmerzmittel sowie als Medikament bei Entzündungserkrankungen offenbart.

Die antioxidativen Eigenschaften der α-Liponsäure beruhen neben der Fähigkeit Chelate mit Metallionen zu bilden sowie direkt Radikale zu eliminieren insbesondere auf der Funktion als starkem Reduktionsmittel. Um diese Reaktion intrazellulär auszuführen, muss α-Liponsäure selbst in reduzierter Form, als Dihydroliponsäure vorliegen. Die Überführung von (disulfidischer) α-Liponsäure mittels Reduktion in die Dithiolform der Dihydroliponsäure verbraucht seinerseits reduzierende Äquivalente, wobei dieser Vorgang u.a. durch das Enzym Glutahionreduktase katalysiert wird (Gen Pharmacol 1997;29:315-331). Dies stellt offenbar die Ursache für die hinsichtlich der Thiolrestitution bislang unbefriedigende Wirkung der Substanz dar.

Die DE 44 20 102 A1 beschreibt eine Arzneimittelkombination aus α-Liponsäure und herz-kreislaufaktiven Substanzen, insbesondere einem organischen Nitrat, einem Calcium-Antagonisten, ACE-Hemmer oder Oxyfedrin. Die Arzeimittelkombination soll zur Behandlung von Herz-Kreislauferkrankungen sowie diabetisch bedingten Erkrankungen eingesetzt werden.

Ambroxol, d.h. trans-4- (2-Amino-3,5-dibromobenzylamino) -cyclohexanhydrochlorid wird in verschiedenen Darreichungsformen bei Lungen- und Bronchialerkrankungen als schleimlösendes Medikament eingesetzt (WO 96 33704, GB 2239242, WO 01 05378). Darüber hinaus ist die Verwendung bei Hyperurikämie aus der DE 35 30 761 bekannt. Die Wirkung des Ambroxol als Mukolytikum beruht sowohl auf einer Stimulation der Surfactantproduktion der Bronchialzellen als auch insbesondere auf der Fähigkeit, freie Radikale zu eliminieren (Respir Med 1998;92:609-23). Diese hierauf basierende antioxidative Aktivität der Substanz wurde hauptsächlich an pulmonalen Zellen (Pharmacol 1999;59:135-141) aber auch im Rahmen von entzündlichen Mechanismen nachgewiesen (Inflamm Res 1999;48:86-93). Weiterhin ist bekannt, dass in vitro durch den Zusatz von Ambroxol in hohen Dosen regulatorische Enzyme des Glutathionstoffwechsels direkt beeinflusst sowie peroxidative Prozesse gehemmt werden (Arch Vet Pol 1992;32:57-66).

Hemmer des Angiotensin-Converting Enzymes (Angiotensin-Converting Enzyme Inhibitors, ACE-Hemmer) werden mit großem Erfolg bei der Behandlung einer breiten Palette kardiovaskulärer Erkrankungen eingesetzt. Die Ursache der hier genutzten blutdrucksenkenden Wirkung beruht auf der Hemmung der Umwandlung von Angiotensin I in Angiotensin II. Darüber hinaus wurden ACE-Hemmer auch als Effektoren des Glutathionstoffwechsels beschrieben. Neben Untersuchungen zu diesbezüglichen Effekten bei Herz-Kreislauf- und Gefäßerkrankungen (J Cardiovasc Pharmacol 2000;36:503-509) wurden allgemeine Regulationsprinzipien untersucht (Clin Nephrol 1997;47:243-247). Zu unterscheiden sind hier die Wirkungen SH-Gruppen tragender ACE-Hemmer wie z.B. Captopril (1-[(2S)-3-Mercapto-2-methylpropionyl]-L-prolin) von SH-freien ACE-Hemmern wie z.B. Enalapril (1-{N-[(S)-1-Ethoxycarbonyl-3-phenylpropyl)-L-alanyly-L-prolin). Erstere reagieren direkt als Radikalfänger antioxidativ während SH freie ACE-Inhibitoren dazu primär nicht in der Lage sind. Beiden Gruppen gemeinsam ist die Beeinflussung des Glutathionredoxzyklusses über die Regulation der Glutathionreduktase und Glutathionperoxidase sowie weiterhin der Superoxiddismutase (Am J Physiol Regulatory Integrative Comp. Physiol.2000;278:572-577).

Aufgabe der vorliegenden Erfindung war es daher, neuartige, thiolreaktive Substanzen enthaltende Arzneimittel zur verbesserten Stabilisierung eines geschädigten Thiol-Disulfidstatus, insbesondere bei Niereninsuffizienz im Rahmen der Nierenersatztherapie, und zur Restitution der dadurch ausgelösten Funktionsverluste bereitzustellen.

Diese Aufgabe wird durch das erfindungsgemäße Arzneimittel mit den Merkmalen des Anspruchs 1 gelöst. In Anspruch 13 wird die Verwendung der Wirkstoffe zur Herstellung eines Arzneimittels beschrieben. Die weiteren abhängigen Ansprüche zeigen jeweils vorteilhafte Weiterbildungen auf.

Erfindungsmäßig werden dabei Effektoren des Glutathionmetabolismus in Kombination mit α-Liponsäure, deren Salze und/oder deren Prodrugs eingesetzt.

Überraschenderweise konnte gezeigt werden, daß durch die Applikation der erfindungsgemäß zur Anwendung kommenden Kombination von α-Liponsäure und einem Effektor des Glutathionmetabolismus eine Normalisierung des primär verringerten Thiolstatus von Immunzellen erfolgte. Die thiolstabilisierende Wirkung der Kombinationen überstieg dabei die der alleinigen Verwendung von α-Liponsäure oder der jeweiligen Effektoren nicht nur regelmäßig, vielmehr konnten auch superadditive Effekte nachgewiesen werden. Die Restitution des Thiolstatus erfaßte dabei sowohl intrazelluläre Thiole als auch membrangebundene SH-Gruppen und ist somit Ausdruck einer komplexen biologischen Regulation. Dieses Phänomen beruht darauf, daß die Effektoren des Glutathionstoffwechsels einerseits intermediär entstehende freie Radikale eliminieren und andererseits die Verfügbarkeit reduzierender Äquivalente für die Umwandlung der α-Liponsäure aus disulfidischer in reduzierte Form erhöhen und somit die Syntheseinduzierende Wirkung der α-Liponsäure auf den Thiol-Disulfidstatus verbessern.

Darüber hinaus wurde deutlich, daß eine thiolsteigernde Wirkung der Kombination von Effektoren des Glutathionmetabolismus und α-Liponsäure nur bei primär thioldefizienten Immunzellen auftrat. Gesunde Immunzellen, welche keine Alteration des Thiol-Disulfidstatus aufwiesen, reagierten nicht mit einer weiteren Steigerung der SH-Konzentration.

Die Restitution des Thiolstatus der Immunzellen wurde begeleitet von einer Normalisierung funktioneller Parameter. Dies betraf insbesondere die immunmodulatorischen Effekte im Rahmen der Aktivierbarkeit von T-Lymphozyten.

Weiterhin konnte gezeigt werden, daß die erfindungsmäßig zur Anwendung kommenden Kombinationen den Thiol-Disulfidstatus von weiteren Immunzellen wie den Peritoneal-makrophagen dialysepflichtiger Patienten stabilisierten. Die Peritonealmakrophagen wiesen vor der Behandlung mit α-Liponsäure / Ambroxol bzw. α-Liponsäure / ACE-Hemmern neben einem defizienten Thiolstatus einen nahezu vollständigen Verlust ihrer Phagozytosefunktion sowie eine schwere Störung der Differenzierung und Zytokinsynthese auf, welche als ursächlich für die hohen Infektionsraten bei diesen Patienten beschrieben sind. Diese Funktionsverluste konnten durch die Zugabe der erfindungsgemäß benannten Kombinationen aufgehoben werden.

Besonders geeignet ist dieses Arzneimittel im Rahmen der Nierenersatztherapie sowie weiteren Krankheitsbildern, bei denen eine Störung des Thiol-Disulfid-Status der Immunzellen auftritt. Dabei kann die Behandlung gleichzeitig, in getrennten Formulierungen oder auch zeitlich abgestuft erfolgen.

Die erfindungsgemäß zur Anwendung kommenden Kombinationspräparate können in den üblichen pharmakologischen Darreichungsformen oder als Instillat sowie prophylaktisch als auch therapeutisch verabreicht werden. Die effektive Dosis ist dabei fallbezogen zu ermitteln. Bevorzugt liegt sie bei der humanmedizinischen Applikation beim Patienten zwischen 30 und 1200 mg/d, besonders bevorzugt zwischen 200 und 600 mg/d.

In einer Variante wird als Effektor des Glutathionmetabolismus Ambroxol der allgemeinen Formel I, 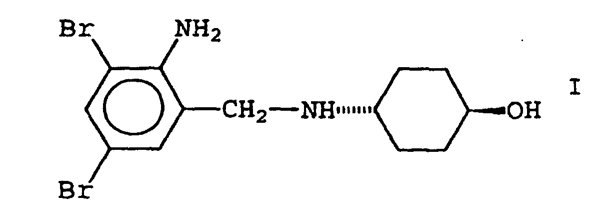 dessen Salz und/oder dessen Prodrug verwendet. Die Dosis von Ambroxol für die humanmedizinische Applikation liegt dabei bevorzugt zwischen 7,5 und 90 mg/d, besonders bevorzugt zwischen 60 und 75 mg/d.

In einer weiteren Variante wird als Effektor des Glutathionmetabolismus ein Inhibitor des Angiotensin-Converting-Enzyms (ACE-Hemmer) verwendet. Hier liegt die bevorzugte Dosis für die humanmedizinische Applikation zwischen 0,2 und 20 mg/d.

Als ACE-Hemmer können dabei z.B. folgende Verbindungen eingesetzt werden:
A) 1-[(2S)-3-Mercapto-2-methylpropionyl)-L-prolin (Captopril) der Formel II 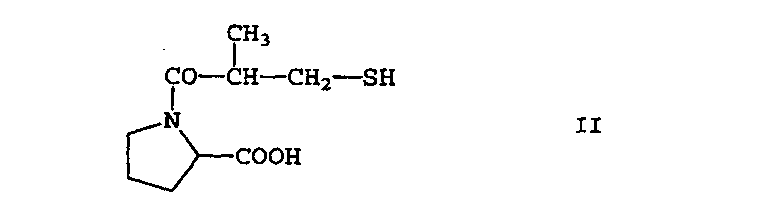
B) 1-{N-[(S)-1-Ethoxycarbonyl-3-phenylpropyl]-Lalanyl}-L-prolin (Enalapril) der Formel III 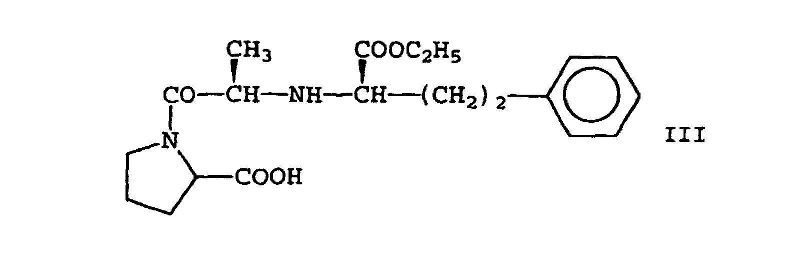
C) (2S,3aS,6aS)-1-{(S)-N-((S)-1-Ethoxycarbonyl-3-phenylpropyl]-alanyl}-octahydrocyclopenta[b]-pyrrol-2-carbonsäure (Ramipril) der Formel IV

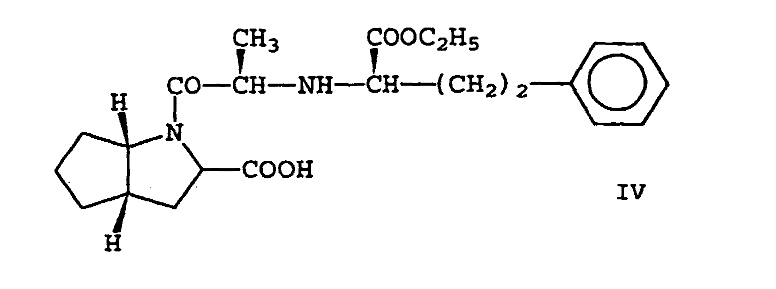

Die Arzneimittel können dabei oral oder auch parenteral verabreicht werden.

Zusätzlich kann das Arzneimittel gängige Additive enthalten. Hierzu zählen z.B. wäßrige Lösungsmittel, Stabilisatoren, Suspensions-, Dispersions- und Benetzungsmittel.

Das Arzneimittel kann in beliebiger Formulierung hergestelllt werden. Beispielsweise gehören hierzu Lösungen, Granulate, Pulver, Emulsionen, Tabletten und/oder Filmtabletten.

Erfindungsgemäß wird ein Effektor des Glutathionmetabolismus zusammen mit α-Liponsäure, deren Salz und/oder deren Prodrugs zur Herstellung eines Arzneimittels zur Behandlung einer Störung des Thiol-Disulfid-Status von Immunzellen im Rahmen der Nierenersatztherapie, verwendet.

Ebenso kann ein Effektor des Glutathionmetabolismus zusammen mit α-Liponsäure, deren Salz und/oder deren Prodrugs zur Herstellung eines Arzneimittels zur immunmodulatorischen, abwehrsteigernden und/oder entzündungshemmenden Behandlung eingesetzt werden.

Die Komponenten des Kombinationspräparats können dabei sowohl in einer einzigen Formulierung als auch in getrennten Formulierungen vorliegen.

Die erfindungsmäßige Verwendung der Kombination von α-Liponsäure und Effektoren des Glutathionstoffwechsels wird anhand der folgenden Beispiele und Figuren näher beschrieben.

### Beispiel 1

### Einfluß auf den zellulären Thiolstatus peripherer Immunzellen des Menschen

Periphere Immunzellen von gesunden Spendern (n=9) wurden mittels Dichtegradientenzentrifugation aus dem peripheren Blut isoliert. Die Hauptfraktion der resultierenden Gesamtpopulation mononukleärer Zellen stellen dabei regelmäßig Lymphozyten mit einem spenderabhängigen relativen Anteil von ca. 90% dar. 10% der mononukleären Zellen werden durch Monozyten repräsentiert.

Die erhaltenen mononukleären Zellen wurden in speziellen Zellkulturmedien aufgenommen und in einem Begasungsbrutschrank bei 37°C, einer relativen Luftfeuchte von 98% und 5% relativem Luft-CO₂-Gehalt inkubiert. Der Stoffwechsel der primär ruhenden Immunzellen wurde mittels mitogener Stimulation (0,5 µg/ml Phytohämagglutinin) aktiviert. Um den Einfluß der erfindungsgemäß zur Anwendung kommenden Kombinationen auf den Thiolstatus thioldefizienter Immunzellen zu prüfen, wurden diese artifiziell thioldepletiert. Dies erfolgte durch Kultivierung in thioldefizienten Medien (RPMI 1603) nach erprobten Verfahren. Vergleichskulturen unter Verwendung von Vollmedien (RPMI 1640) dienten der Definition des unter Kulturbedingungen bestmöglichen Normalwertes.

Die Bestimmung des interzellulären Thiolgehaltes auf Einzelzellebene erfolgte unter Verwendung von 5-Chloromethylfluoresceindiacetat (CMFDA) in der Durchflußzytofluorimetrie.

Primär nicht fluorogenes CMFDA wird dabei passiv von der Zelle aufgenommen. Über den Chlormethylrest erfolgt eine Bindung an zytoplasmatische Thiolgruppen. Nach Abspaltung der Acetatreste durch unspezifische zelluläre Esterasen wird dieser, nun zellmembranimpermeabele Komplex bei einer Exitationswellenlänge λₑₓ = 490 nm mit einer Emissionswellenlänge λₑₘ = 520 nm fluorogen. Die mittlere Fluoreszenzintensität der Probe (10.000 Zellen) ist der Konzentration der intrazellulären Thiolgruppen direkt proportional.

Die Expression membrangebundener Thiolgruppen wurde ebenfalls durchflußzytofluorimetrisch ermittelt. Hierbei wurde Chloromethyltetramethylrhodamin (CMTMR) unter den Bedingungen eines blockierten Membranpotentials sowie einer gehemmten Diffusionskapazität der Zellen als Thiolkonjugat eingesetzt. Die Fluoreszenzintensität der gebunden Fluorochrommoleküle an der Zellmembran ist dabei wiederum proportional der Menge der Thiolgruppen an der Zelloberfläche.

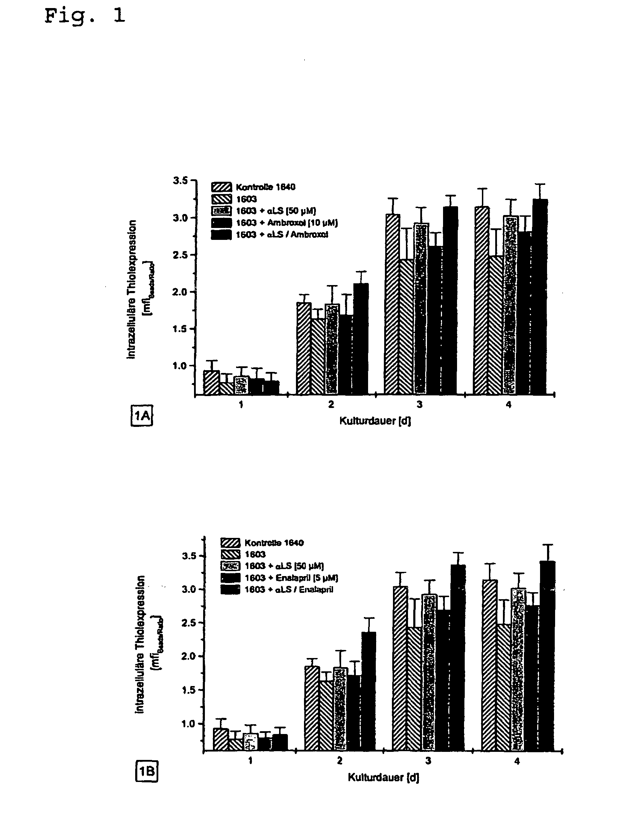

In Fig. 1 ist die Wirkung der Kombination von α-Liponsäure und Ambroxol (Fig. 1a) sowie α-Liponsäure und Enalapril (Fig. 1b) auf die intrazelluläre Thiolexpression von Lymphozyten dargestellt. Die folgende Tabelle zeigt die Ergebnisse der Kombination von α-Liponsäure und Captopril. Die Daten sind als Verhältnis der zellulären Fluoreszenzintensität zu jeweils parallel analysierten Kalibrierungspartikeln (Beads) dargestellt. Die Wirkstoffkonzentration der jeweiligen Kombination ist identisch mit den Konzentrationen der einzelnen Komponenten.

| intrazelluläre Thiolexpression [mfi_{Beads/Ratio}] | | | | |
|---|---|---|---|---|
| Kulturdauer [d] | Kontrolle | α-Liponsäure [50 µM] | Captopril [10 µM] | α-Liponsäure + Captopril |
| 0 | 2,88 ± 0,20 | 2,88 ± 0,20 | 2,88 ±0,20 | 2,88 ± 0,20 |
| 1 | 2,31 ± 0,20 | 2,81 ±0,23 | 2,80 ± 0,21 | 2,89 ± 0,31 |
| 2 | 1,98 ± 0,16 | 2,76 ± 0,50 | 2,76 ± 0,22 | 2,92 ± 0,32 |
| 3 | 1,63 ± 0,15 | 2,63 ± 0,60 | 2,49 ± 0,26 | 2,88 ± 0,41 |
| 4 | 1,30 ± 0,16 | 2,41 ± 0,40 | 2,21 ± 0,36 | 2,91 ± 0,39 |
| 6 | 1,10 ± 0,13 | 2,23 ± 0,50 | 1,83 ± 0,33 | 2,93 ± 0,35 |
| 8 | 0,95 ± 0,10 | 2,02 ± 0,30 | 1,02 ± 0,39 | 2,93 ± 0,41 |
| 10 | 0,81 ± 0,10 | 1,89 ± 0,30 | 0,91 ± 0,46 | 2,90 ± 0,45 |
| 12 | 0,69 ± 0,10 | 1,86 ± 0,68 | 0,76 ± 0,49 | 2,88 ± 0,49 |
| 14 | 0,65 ± 0,08 | 1,83 ± 0,60 | 0,75 ± 0,56 | 2,86 ± 0,47 |

Periphere Immunzellen wurden über einen Zeitraum von 4 Tagen unter normalen (Kontrolle1640) bzw. thioldefizienten Bedingungen (1603) zur Induktion einer 10-20%igen Thiolreduktion kultiviert. Wie in 1a gezeigt, resultiert die Zugabe von Ambroxol in Kombination mit α-Liponsäure beginnend nach 48 Stunden Behandlungsdauer in einem vollständigen Ausgleich des intrazellulären Thioldefizites. Unter Verwendung der Kombination von α-Liponsäure und dem SH-freien ACE-Hemmer Enalapril sowie dem SH-tragenden ACE-Hemmer Captopril waren diese Effekte noch quantitativ verstärkt sowie in der Zeitkinetik bereits nach 24 Stunden nachweisbar. Weder durch α-Liponsäure allein noch durch die Einzelapplikation der Effektoren war ein vollständiger Ausgleich des Thioldefizites möglich.

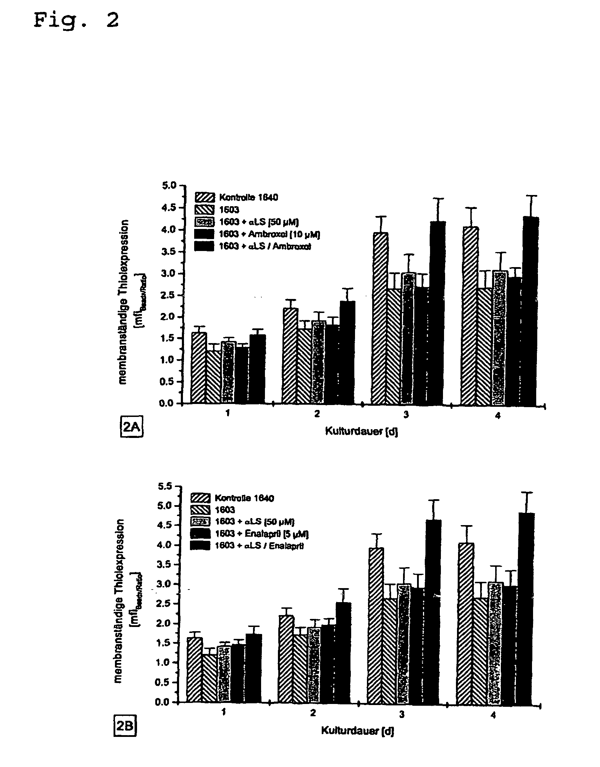

Die mit diesem experimentellen Ansatz erhaltenen Resultate für den Einfluss der erfindungsmäßig benannten Kombinationen auf die Expression zellmembranständiger Thiole sind in Fig. 2 für die Kombination von α-Liponsäure und Ambroxol (Fig. 2a) sowie α-Liponsäure und Enalapril (Fig. 2b) dargestellt; die folgende Tabelle gibt die Ergebnisse der Kombination von α-Liponsäure und Captopril wieder.

| membranständige Thiolexpression [mfi_{Beads/Ratio}] | | | | |
|---|---|---|---|---|
| Kulturdauer [d] | Kontrolle | α-Liponsäure [50 µM] | Captopril [10 µM] | α-Liponsäure + Captopril |
| 0 | 2,37 ± 0,45 | 2,37 ± 0,45 | 2,37 ± 0,45 | 2,37 ± 0,39 |
| 1 | 2,79 ± 0,50 | 2,65 ± 0,39 | 2,63 ± 0,39 | 2,38 ± 0,38 |
| 2 | 2,35 ± 0,45 | 2,43 ± 0,52 | 2,54 ± 0,41 | 2,42 ± 0,41 |
| 3 | 1,98 ± 0,43 | 2,31 ± 0,36 | 2,52 ± 0,38 | 2,49 ± 0,46 |
| 4 | 1,63 ± 0,43 | 2,26 ± 0,20 | 2,50 ± 0,41 | 2,39 ± 0,52 |
| 6 | 1,10 ± 0,46 | 2,19 ± 0,13 | 2,46 ± 0,50 | 2,40 ± 0,50 |
| 8 | 0,98 ± 0,31 | 1,93 ± 0,20 | 2,01 ± 0,39 | 2.40 ± 0,53 |
| 10 | 0,96 ± 0,32 | 1,63 ± 0,16 | 1,68 ± 0,29 | 2,36 ± 0,52 |
| 12 | 0,95 ± 0,33 | 1,32 ± 0,21 | 1,02 ± 0,51 | 2,38 ± 0,49 |
| 14 | 0,98 ± 0,33 | 1,34 ± 0,20 | 0,99 ± 0,46 | 2,36 ± 0,55 |

Unter der Behandlung mit der Kombination α-Liponsäure und Ambroxol kam es wiederum beginnend nach 48 Stunden zu einer signifikanten Verbesserung der membranständigen Thiolexpression. Besonders auffällig war hier, dass die Gabe der Einzelsubstanzen zu keinem Zeitpunkt einen signifikanten Einfluss zeigte. Die Zugabe der Kombination von α-Liponsäure und den jeweiligen ACE-Inhibitoren resultierte sowohl bei Enalapril als auch bei Captopril in einem superadditiven Effekt.

### Beispiel 2

### Einfluß auf den zellulären Aktivierungsstatus peripherer T-Lymphozyten des Menschen

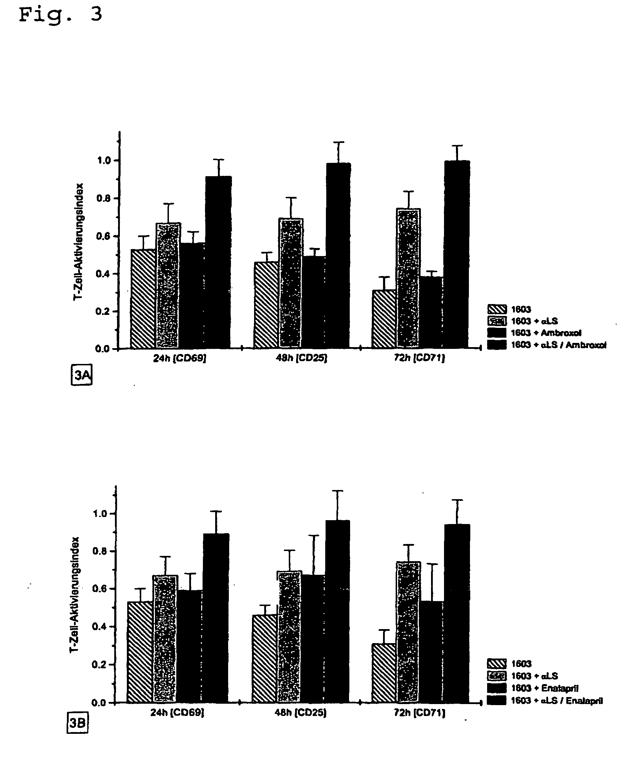

In dem unter Beispiel 1 beschriebenen Kultivierungsansatz wurden humane T-Lymphozyten mit 1,0 µg/ml Phytohämagglutinin stimuliert. innerhalb einer Kulturdauer von 72 Stunden wurden spezifische Marker der zellulären Aktivierung zytofluorimetrisch durch Detektion mittels monoklonaler Antikörper quantitativ nachgewiesen. Untersucht wurde der Einfluss der erfindungsgemäß zur Anwendung kommenden Kombinationen auf die Aktivierungsmarker CD69 (frühes Aktivierungsantigen), CD25 (intermediäres Aktivierungsantigen) und CD71 (spätes Aktivierungsantigen ) von T-Lymphozyten. In Fig. 3 ist die Wirkung der Kombination von α-Liponsäure und Ambroxol (Fig. 3a) sowie α-Liponsäure und Enalapril (Fig. 3b) auf den Aktivierungsindex von T-Lymphozyten dargestellt. Verglichen mit normalen T-Lymphozyten (Aktivierungsindex = 1,0) ist bei thioldefizienten Zellen eine, die zelluläre Funktionstörung belegende, deutliche Verringerung der Aktivierbarkeit zu verzeichnen. Nach Zugabe von α-Liponsäure tritt der bekannte Effekt einer leichten Verbesserung der zellulären Aktivierbarkeit auf, welche jedoch in keinem Fall die signifikante Abweichung von der normalen Kontrollgruppe behebt. Ambroxol zeigt keinen Einfluss auf einen der drei Akitivierungsmarker; der ACE-Hemmer Enalapril ist nur im Falle der Effektuierung des CD25-Antigens der α-Liponsäure gleichwertig. Demgegenüber war sowohl bei der kombinierten Anwendung von α-Liponsäure und Ambroxol als auch α-Liponsäure und Enalapril eine Anhebung des T-Zell-Aktivierungsindex in den Normalbereich nachweisbar. Dieser Effekt wurde bei frühen, intermediären und späten Aktivierungsmarkern beobachtet. Es kann somit geschlussfolgert werden, dass die durch die kombinierte Verwendung von α-Liponsäure und den jeweiligen Effektoren des Glutathionstoffwechsels vermittelte Normalisierung des zellulären Thiolstatus einhergeht mit einer Restitution der zellulären Funktionalität.

### Beispiel 3

### Einfluss auf den zellulären Thiolstatus von Peritonealmakrophagen im Rahmen der Nierenersatztherapie

Peritonealmakrophagen wurden aus dem Effluat der Peritonealdialyse hochgradig niereninsuffizienter Patienten isoliert, in Zellkulturmedium aufgenommen und in einem Begasungsbrutschrank bei 37°C, einer relativen Luftfeuchte von 98% und 7,5% relativem Luft-CO₂-Gehalt inkubiert. Um den Einfluß der erfindungsgemäß zur Anwendung kommenden Kombinationen auf den Thiolstatus der Peritonealmakrophagen zu prüfen, wurden jeweils eine Fraktion mit α-Liponsäure, den Effektoren des Glutathionmetabolismus Ambroxol oder dem ACE-Hemmer Enalapril bzw. mit der Kombination von α-Liponsäure / Ambroxol oder α-Liponsäure / Enalapril behandelt, während jeweils eine weitere Fraktion als unbehandelte Kontrolle geführt wurde.

Die Bestimmung des zellulären Thiolstatus erfolgte mittels der unter 1. beschriebenen Meßmethode. Die Membranexpression von Thiolen wurde anhand der mittleren Fluorezenzintensität(mfi) der Probe (3000 Zellen/Messung) nach Kopplung an ein Chlormethyl-Fluorochromderivat bestimmt.

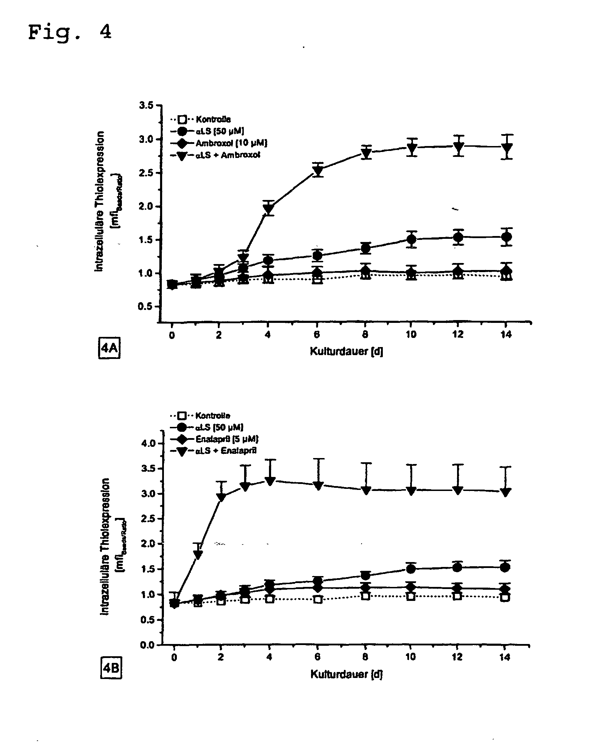

In der Fig. 4 ist der Effekt der Kombination von α-Liponsäure und Ambroxol (Fig. 4a) sowie α-Liponsäure und Enalapril (Fig. 4b) in der Zeitkinetik über 14 Tage dargestellt (n=12).

Unter Zusatz der Monosubstanzen war wiederum nur ein Anstieg der zellulären Thiolexpression unter Verwendung von α-Liponsäure zu beobachten, während Ambroxol und der ACE-Hemmer keinen Effekt zeigten. Demgegenüber war unter der Kombination von α-Liponsäure und Ambroxol beginnend nach 72 Stunden ein deutlicher Anstieg der zellulären Thiolexpression nachweisbar, der nach 4 Tagen Behandlungsdauer einen superadditiven Effekt sowie nach 8 Tagen ein Maximum erreichte, welches die Ausgangs- bzw. Kontrolldaten um das Dreifache überstieg (Fig. 4a). Die Kombination von α-Liponsäure und einem ACE-Hemmer (Fig. 4b) resultierte in einer ähnlichen, jedoch nochmals deutlich verkürzten Zeitkinetik. Ein Maximum der superadditiven Wirkung war hier bereits nach 48 - 72 Stunden Behandlungsdauer erreicht.

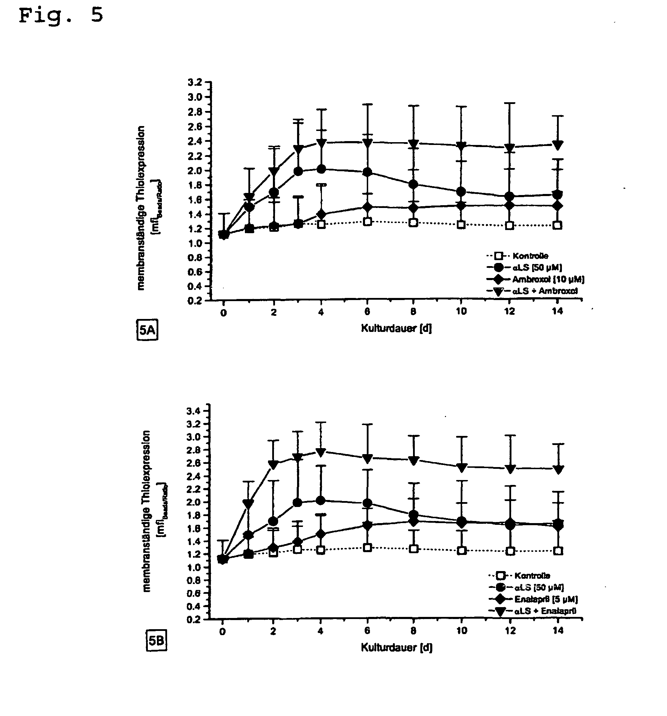

In Fig. 5 ist die Wirkung der Kombination von α-Liponsäure und Enalapril (Fig. 5b) auf die membranständige Thiolexpression von Peritonealmakrophagen in der oben beschriebenen Versuchsanordnung dargestellt. Die Membranexpression von Thiolen wurde anhand der mittleren Fluorezenzintensität(mfi) der Probe (3000 zellen/Messung) nach Kopplung an ein Chlormethyl-Fluorochromderivat bestimmt. Im Vergleich mit den Ergebnissen der intrazellulären Thiolexpression ist hier ein sehr deutlicher Effekt der alleinigen Gabe von α-Liponsäure zu verzeichnen, der jedoch nach 4 Behandlungstagen wieder aufgehoben ist. Im Gegensatz dazu bewirkt die kombinierte Applikation von α-Liponsäure und Ambroxol (Fig. 5a) bzw. α-Liponsäure und einem ACE-Hemmer (Fig. 5b) sowohl einen primär deutlicheren als auch über den Beobachtungszeitraum hinweg stabilen, superadditiven Anstieg der membranständigen Thiolexpression.

### Beispiel 4

### Einfluss auf die Phagozytosefähigkeit von Peritonealmakrophagen

Um eine Charakterisierung der Peritonealmakrophagen hinsichtlich ihrer originären Funktionen zu ermöglichen, wurde die Phagozytosefähigkeit als Messgröße ausgewählt.

Peritonealmakrophagen wurden analog des unter Beispiel 3 beschriebenen Vorgehens isoliert und ex vivo kultiviert. Die Bestimmung der Phagozytoseleistung erfolgte durch einen zytofluorimetrischen Test auf Einzelzellebene. Dabei wurden die Makrophagen mit opsonierten und fluorochrommarkierten Bakterien cokultiviert. Die Menge der in einem definierten Zeitraum aufgenommenen Bakterien wurde quantitativ über die Fluoreszenzintensität in den Makrophagen erfaßt und galt als Maß für deren Phagozytosekapazität.

Der Einfluß der erfindungsgemäß zur Anwendung kommenden Kombinationen auf die Phagozytosefähigkeit der Peritonealmakrophagen nach einer Behandlungsdauer von 6 Tagen ist in folgender Tabelle dargestellt.

| | Phagozytoserate [mfi / 10.000 Zellen] |
|---|---|
| Kontrolle | 371 ± 39 |
| | |
| α-Liponsäure [50 µM] | 687 ± 59 |
| | |
| Ambroxol [10 µM] | 501 ± 52 |
| | |
| α-Liponsäure + Ambroxol | 1.398 ± 286 (p<0.05) |
| | |
| Enalapril [5 µM] | 567 ± 59 |
| | |
| α-Liponsäure + Enalapril | 1.698 ±241 (p<0.05) |
| | |
| Captopril [10 µM] | 653 ± 43 |
| | |
| α-Liponsäure + Captopril | 1.589 ± 176 (p<0.05) |

Nach Inkubation mit α-Liponsäure, Ambroxol bzw. Enalapril war die Phagozytoserate gegenüber der unbehandelten Kontrolle um den Faktor 1,85 (α-Liponsäure), 1,35 (Ambroxol) bzw. 1,53 (Enalapril) erhöht. Demgegenüber konnte unter Verwendung der Kombination von α-Liponsäure und Ambroxol eine Steigerung der Phagozytoserate um den Faktor 3,7, bei Verwendung der Kombination von α-Liponsäure und einem ACE-Hemmer um den Faktor 4,6 (Enalapril) bzw. 4,3 (Captopril) erreicht werden.

Darüber hinaus konnte eine direkte Korrelation zwischen der Phagozytoserate und dem intrazellulären Thiolgehalt der Peritonealmakrophagen für die Kombination von α-Liponsäure und Ambroxol (r=0,79; p<0,01), α-Liponsäure und Captopril (r=0,86; p<0,01) sowie α-Liponsäure und Enalapril (r=0,82; p<0,01) nachgewiesen werden.

### Beispiel 5

### Einfluss auf den Differenzierungs- und Aktivierungsgrad sowie die Zytokinsynthese von Peritonealmakrophagen

Peritonealmakrophagen wurden von Patienten unter Nierenersatztherapie nach den unter Beispiel 3 beschriebenen Verfahren isoliert und in Gegenwart der erfindungsgemäß benannten Kombinationen von α-Liponsäure und Effektoren des Gluatathionmetabolismus kultiviert. Nach 6 Tagen Inkubation wurde der Grad der Differenzierung der Peritonealmakrophagen über die Expression der Zelloberflächenantigene CD15 und CD11c sowie der Grad der zellulären Aktivierung über die Koexpression der Aktivierungsantigene CD69 auf CD15-positiven Zellen sowie CD71 auf CD11c-positiven Zellen zytofluorimetrisch bestimmt.

Die Ergebnisse sind in folgender Tabelle zusammengestellt.

| | CD15 | CD11c | CD15/69 | CD11c/71 |
|---|---|---|---|---|
| Kontrolle | 1,0 | 1,0 | 1,0 | 1,0 |
| α-Liponsäure [50 µM] | 1,18 ± 0,16 | 1,21 ± 0,11 | 1,09 ± 0,08 | 1,08 ± 0,09 |
| Ambroxol [10 µM] | 0,98 ± 0,13 | 1,01 ± 0,09 | 0,98 ± 0,11 | 0,96 ± 0,1 |
| α-Liponsäure + Ambroxol | 1,29 ± 0,21 | 1,65 ± 0,21 | 1,49 ±0,13 | 1,83± 0,14 |
| Enalapril [5 µM] | 1,21 ± 0,22 | 1,23 ± 0,22 | 1,19 ± 0,12 | 1,10 ± 0,14 |
| α-Liponsäure+ Enalapril | 2,12 ± 0,16 (p<0.05) | 1,99 ± 0,15 (p<0.05) | 1,69 ± 0,2 | 1,58 ± 0,12 |
| Captopril [10 µM] | 1,19 ± 0,14 | 1,26 ± 0,24 | 1,69 ± 0,21 | 1,52 ± 0,16 |
| α-Liponsäure + Captopril | 2,25 ± 0,2 (p<0.05) | 2,63 ± 0,23 (p<0.05) | 1,74 ± 0,19 | 1,61 ± 0,18 |

Es konnte gezeigt werden, dass die Expression der Reifungsmarker CD15 und CD11c unter Verwendung der Kombination von α-Liponsäure und Ambroxol deutlich, unter Verwendung der Kombination α-Liponsäure und ACE-Hemmer signifikant anstieg. Darüber hinaus war eine deutliche Zunahme der Aktivierungsantigene CD69 bzw. CD71 auf den jeweiligen Zellpopulationen nachweisbar. Die Applikation der Monosubstanzen hatte keinen oder nur marginalen Einfluss auf den Differenzierungs- und Aktivierungsgrad von Peritonealmakrophagen.

Parallel hierzu wurden in diesem experimentellen Ansatz die Zellkulturüberstande gewonnen und die darin enthaltenen, von den Peritonealmakrophagen synthetisierten und sekretierten Zytokine Interleukin-6 (IL-6) sowie Interleukin-1 Rezeptorantagonist (IL-1ra) bestimmt. Die Analyse erfolgte unter Verwendung der Enzymimmunoassay-Technik mit standardisierten Meßsystemen und ist in der folgenden Tabelle dargestellt, die die Effekte von α-Liponsäure und Effektoren des zellulären Glutathionmetabolismus auf die Zytokinsynthese von Peritonealmakrophagen nach einer Behandlungsdauer von 6 Tagen (n=10) zeigt.

| | IL-6 [ng/10⁶ Zellen] | IL-1ra [ng/10⁶ Zellen] |
|---|---|---|
| Kontrolle | 53.1 ± 8.9 | 115.2 ± 23.4 |
| | | |
| α-Liponsäure [50 µM] | 46.9 ± 6.7 | 119.8 ± 19.5 |
| | | |
| Ambroxol [10 µM] | 51.8 ± 8.1 | 118.6 ± 21.3 |
| | | |
| α-Liponsäure + Ambroxol | 31.5 ± 9.2 (p<0.05) | 126.8 ± 15.3 (p<0.05) |
| | | |
| Enalapril [5 µM] | 41.7 ± 7.3 | 121.1 ± 16.9 |
| | | |
| α-Liponsäure + Enalapril | 22.3 ± 8.1 (p<0.05) | 139.8 ± 22.1 (p<0.05) |
| | | |
| Captopril [10 µM] | 42.9 ± 7.7 | 129.4 ± 25.1 |
| | | |
| α-Liponsäure + Captopril | 28,1 ± 6.1 (p<0.05) | 143.5 ± 18.7 (p<0.05) |

In Gegenwart der Kombination von α-Liponsäure und Ambroxol sowie der Kombination von α-Liponsäure und den unterschiedlichen ACE-Inhibitoren war eine signifikante Reduktion der IL-6 Synthese nachweisbar. Dieser Effekt ging wiederum deutlich über die Summe der durch die Monosubstanzen vermittelten Verringerung hinaus. Die Synthese von IL-Ira wurde unter diesen Bedingungen signifikant induziert. Auch hier war ein superadditiver Einfluss der Kombination von α-Liponsäure und Ambroxol bzw. ACE-Inhibitoren zu verzeichnen.

### Beispiel 6

### Einfluss auf die Stabilität der Thiolrestitution bei Peritonealmakrophagen im Dialysemodell

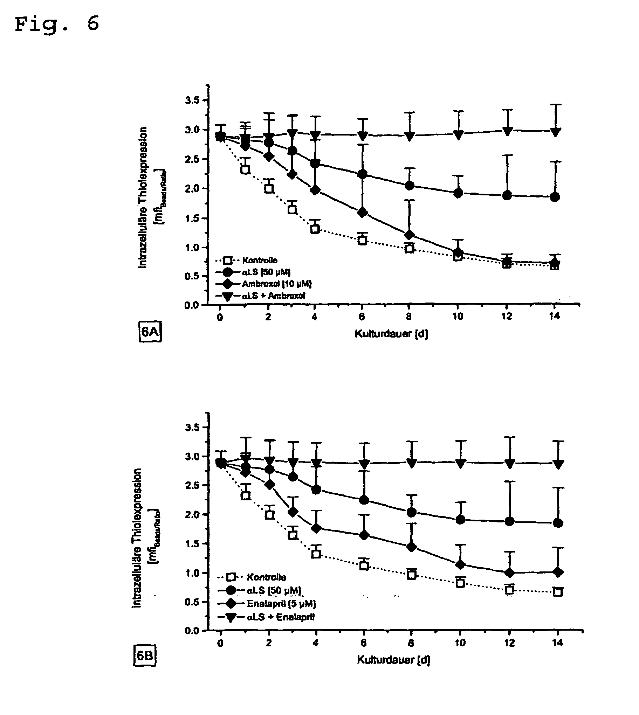

Die unter Beispiel 3 beschriebenen, mittels der erfindungsgemäß zur Anwendung kommenden Kombinationen thiolrestituierten Peritonealmakrophagen wurden nach 6 Tagen diesem Testsystem entnommen und über einen Zeitraum von 14 Tagen in einem Dialysemodell kultiviert. Hierzu wurden die Peritonealmakrophagen auf mobilen Kollagen IV-beschichteten Matrices adaptiert und 3 mal täglich für je 60 Minuten mit herkömmlicher Dialyselösung in Kontakt gebracht. Dieses Modell diente hier zur Induktion eines kombinierten hyperglykämischen/osmotischen Stresses. In Fig. 6 ist die Wirkung der Kombination von α-Liponsäure und Ambroxol (Fig. 6a) sowie α-Liponsäure und Enalapril (Fig. 6b) auf die intrazelluläre Thiolexpression der Peritonealmakrophagen in der Zeitkinetik dargestellt. Die Membranexpression von Thiolen wurde anhand der mittleren Fluorezenzintensität(mfi) der Probe (3000 Zellen/Messung) nach Kopplung an ein Chlormethyl-Fluorochromderivat bestimmt.

Während bei den primär thiolrestituierten, in diesem Dialysemodell unbehandelten Kontrollen innerhalb der ersten 4 Tage ein nahezu lineares Absinken der intrazellulären Thiolkonzentration zu verzeichnen war, resultiert die kombinierte Zugabe von α-Liponsäure und Ambroxol sowie von α-Liponsäure und Enalapril in einem konstanten intrazellulären Thiolstatus auf dem Niveau der primären Restitution. Auch hier ist insbesondere durch α-Liponsäure ein Monoeffekt nachweisbar, der jedoch nur kurz anhaltend ist und nach ca. 4 Tagen im Dialysemodell nur etwa 50% der Wirkung der Kombinationen zeigt.

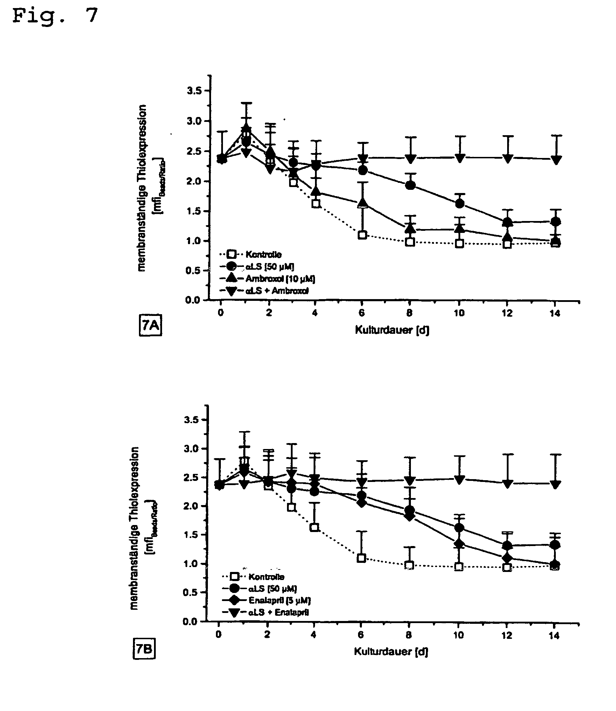

Ein ähnliches Bild bietet sich bei der Betrachtung der in Fig. 7 dargestellten Verläufe der membrangebundenen Thiolexpression. Wiederum werden die durch die primäre Thiolrestitution erhaltenen Quantitäten durch Verwendung der Kombination von α-Liponsäure und Ambroxol (Fig. 7a) bzw. ACE-Hemmern (Fig. 7b) konstant gehalten, während unter Zugabe der Monosubstanzen nur intermediäre (α-Liponsäure) bzw. marginale Effekte (Ambroxol, Enalapril) beobachtet wurden.

Insgesamt machen diese Versuche deutlich, daß die Applikation der Kombination von α-Liponsäure und den Effektoren des Glutathionmetabolismus Ambroxol bzw. ACE-Inhibitoren einen primär massiv geschädigten Thiolstatus in unterschiedlichen Zellsystemen stabilisiert. Durch diese Normalisierung kommt es darüber hinaus zu einer Wiederherstellung zentraler zellulärer immunregulatorischer Funktionen, welche ohne eine solche Behandlung nicht zu verzeichnen ist.

## Patentansprüche

1. Arzneimittel enthaltend einen Effektor des Glutathionmetabolismus zusammen mit α-Liponsäure, deren Salze und/oder deren Prodrugs als Kombinationspräparat zur gleichzeitigen, getrennten oder zeitlich abgestuften Behandlung einer Störung des Thiol-Disulfid-Status im Rahmen der Nierenersatztherapie sowie Krankheitsbildern, bei denen eine Störung des Thiol-Diaulfid-Status von Immunzellen auftritt, wobei als Effektor Ambroxol mit der allgemeinen Formel I, 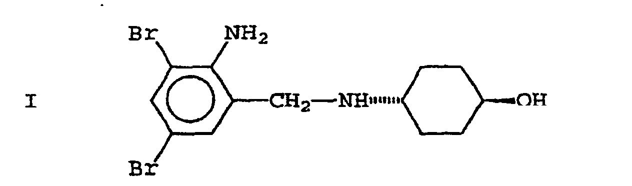 deren Salze und/oder deren Prodrugs verwendet wird.

2. Arzneimittel nach Anspruch 1,
**dadurch gekennzeichnet, dass** die Dosis der α-Liponsäure, deren Salze und/oder deren Prodrugs für die humanmedizinische Applikation beim Patienten zwischen 30 und 1200 mg/d, bevorzugt zwischen 200 und 600 mg/d liegt.

3. Arzneimittel nach Anspruch. 1 oder 2,
**dadurch gekennzeichnet, dass** die Dosis von Ambroxol, deren Salze und/oder deren Prodrugs für die humanmedizinische Applikation beim Patienten zwischen 7,5 und 90 mg/d, bevorzugt zwischen 60 und 75 mg/d liegt.

4. Arzneimittel nach mindestens einem der Ansprüche 1 bis 3,
**dadurch gekennzeichnet, dass** die Verabreichung oral oder parenteral erfolgt.

5. Arzneimittel nach mindestens einem der Ansprüche 1 bis 4,
**dadurch gekennzeichnet, dass** weitere Additive ausgewählt sind aus der Gruppe wässriger Lösungsmittel, Stabilisatoren, Suspensions-, Dispersions- und Benetzungsmittel.

6. Arzneimittel nach mindestens einem der Ansprüche 1 bis 5 in Form einer Lösung, eines Granulats, eines Pulvers, einer Emulsion, einer Tablette und/oder Filmtablette.

7. Verwendung von mindestens einem Effektor des Glutathionmetabolismus zusammen mit α-Liponsäure, deren Salze und/oder deren Prodrugs zur Herstellung eines Arzneimittels zur gleichzeitigen, getrennten oder zeitlich abgestuften Behandlung einer Störung des Thiol-Disulfid-Status im Rahmen der Nierenersatztherapie in Form eines Hämo- oder Peritonealdialyseverfahrens.

8. Verwendung von mindestens einem Effektor des Glutathionmetabolismus zusammen mit α-Liponsäure, deren Salze und/oder deren Prodrugs zur Herstellung eines Arzneimittels zur gleichzeitigen, getrennten oder zeitlich abgestuften Behandlung einer Störung des Thiol-Disulfid-Status im Rahmen von Krankheitsbildern, bei denen eine Störung des Thiol-Disulfid-Status von Immunzellen auftritt, insbesondere zur gleichzeitigen, getrennten oder zeitlich abgestuften immunmodulatorischen, abwehrsteigernden und/oder entzündungshemmenden Behandlung.

9. Verwendung nach Anspruch 7 und 8, **dadurch gekennzeichnet, dass** als Effektor ein Inhibitor des Angiotensin-Converting-Enzyms (ACE-Inhibitor) verwendet wird.

10. Verwendung nach Anspruch 9,
**dadurch gekennzeichnet, dass** die Dosis des Inhibitors des Angiotensin-Converting-Enzyms (ACE-Inhibitor) für die humanmedizinische Applikation beim Patienten zwischen 0,2 und 20 mg/d liegt.

11. Verwendung nach Anspruch 9 oder 10,
**dadurch gekennzeichnet, dass** als ACE-Hemmer 1-[(2S)-3-Mercapto-2-methylpropionyl)-L-prolin (Captopril) der Formel I I 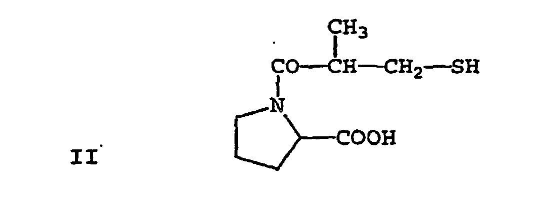 verwendet wird.

12. Verwendung nach Anspruch 9 oder 10,
**dadurch gekennzeichnet, dass** als ACE-Hemmer 1-{N-[(6)-1-Ethoxycarbonyl-3-phenylpropyl]-L-alanyl}-Lprolin (Enalapril) der Formel III 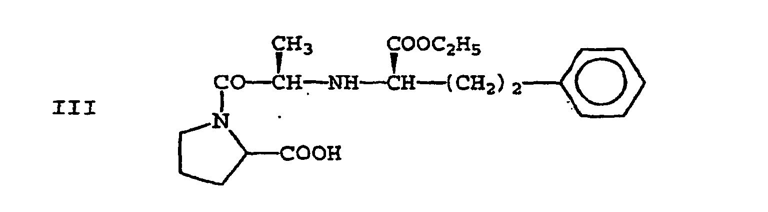 verwendet wird.

13. Verwendung nach Anspruch 9 oder 10,
**dadurch gekennzeichnet, dass** als ACE-Hemmer (2S, 3aS, 6aS)-1-{(S)-N-[(S)-1-Ethoxycarbonyl-3-phenylpropyl] -alanyl} -octahydrocyclopenta [b] -pyrrol-2-carbonsäure (Ramipril) der Formel IV 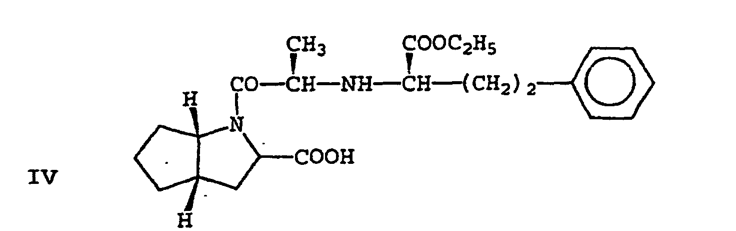 verwendet wird.

14. Verwendung nach mindestens einem der Ansprüche 7 bis 13,
**dadurch gekennzeichnet, dass** der Effektor des Glutathionmetabolismus und die α-Liponsäure, deren Salze und/oder deren Prodrugs in einer einzigen Formulierung vorliegen.

15. Verwendung nach mindestens einem der Ansprüche 7 bis 13,
**dadurch gekennzeichnet, dass** der Effektor des Glutathionmetabolismus und die α-Liponsäure, deren Salze und/oder deren Prodrugs in getrennten Formulierungen vorliegen.

## Claims

1. A medicament comprising an effector of the glutathione metabolism together with α-lipoic acid, its salts and/or its prodrugs as a combination preparation for the simultaneous, separate or sequential treatment of a disturbance of the thiol-disulfide status in kidney replacement therapy, and syndromes in which a disturbance of the thiol-disulfide status of immune cells occurs, where as an effector ambroxole having the general formula I, 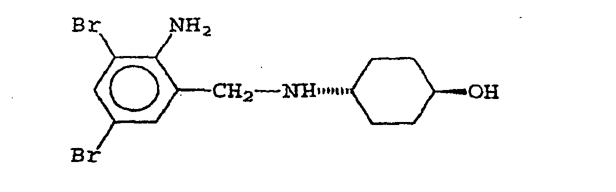 its salts and/or its prodrugs is used.

2. The medicament as claimed in claim 1,
**characterized in that** the dose of α-lipoic acid, its salts and/or its prodrugs for human medicinal administration to the patient is between 30 and 1200 mg/d, preferably between 200 and 600 mg/d.

3. The medicament as claimed in claim 1 or 2,
**characterized in that** the dose of ambroxole, its salts and/or its prodrugs for human medicinal administration to the patient is between 7.5 and 90 mg/d, preferably between 60 and 75 mg/d.

4. The medicament as claimed in at least one of claims 1 to 3, **characterized in that** the administration is carried out orally or parenterally.

5. The medicament as claimed in at least one of claims 1 to 4, **characterized in that** further additives are selected from the group consisting of aqueous solvents, stabilizers, suspending, dispersing and wetting agents.

6. The medicament as claimed in at least one of claims 1 to 5 in the form of a solution, granules, a powder, an emulsion, a tablet and/or film-coated tablet.

7. The use of at least one effector of the glutathione metabolism together with α-lipoic acid, its salts and/or its prodrugs for the production of a medicament for the simultaneous, separate or sequential treatment of a disturbance of the thiol-disulfide status in kidney replacement therapy in the form of a hemo- or peritoneal dialysis process.

8. The use of at least one effector of the glutathione metabolism together with α-lipoic acid, its salts and/or its prodrugs for the production of a medicament for the simultaneous, separate or sequential treatment of a disturbance of the thiol-disulfide status in syndromes in which a disturbance of the thiol-disulfide status of immune cells occurs, in particular for simultaneous, separate or sequential immuno-modulatory, defense-increasing and/or antiinflammatory treatment.

9. The use as claimed in claims 7 and 8,
**characterized in that** the effector used is an angiotensin-converting enzyme inhibitor (ACE inhibitor).

10. The use as claimed in claim 9,
**characterized in that** the dose of the angiotensin-converting enzyme inhibitor (ACE inhibitor) for human medicinal administration to the patient is between 0.2 and 20 mg/d.

11. The use as claimed in claim 9 or 10,
**characterized in that** the ACE inhibitor used is 1-[(2S)-3-mercapto-2-methylpropionyl]-L-proline (captopril) of the formula II.

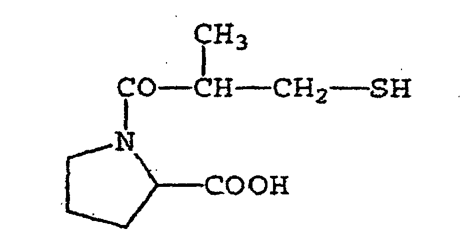

12. The use as claimed in claim 9 or 10,
**characterized in that** the ACE inhibitor used is 1-{N-[(S)-1-ethoxycarbonyl-3-phenylpropyl)-L-alanyl}-L-proline (enalapril) of the formula III.

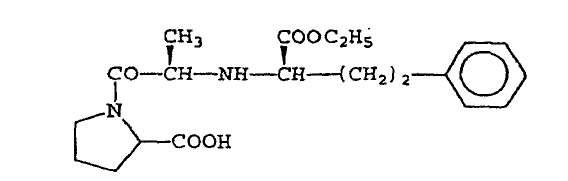

13. The use as claimed in claim 9 or 10,
**characterized in that** the ACE inhibitor used is (2S, 3aS, 6aS)-1-{(S)-N-[(S)-1-ethoxycarbonyl-3-phenylpropyl]-alanyl}-octahydrocyclopenta[b]-pyrrole-2-carboxylic acid (ramipril) of the formula IV.

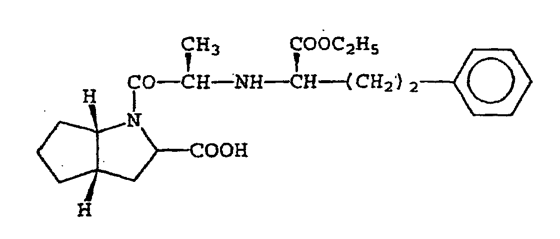

14. The use as claimed in at least one of claims 7 to 13, **characterized in that** the effector of the glutathione metabolism and the α-lipoic acid, its salts and/or its prodrugs are present in a single formulation.

15. The use as claimed in at least one of claims 7 to 13, **characterized in that** the effector of the glutathione metabolism and the α-lipoic acid, its salts and/or its prodrugs are present in separate formulations.

## Revendications

1. Médicament contenant un effecteur du métabolisme du glutathion conjointement avec l'acide α-lipoïque, ses sels et/ou ses promédicaments sous forme de préparation d'association pour le traitement simultané, séparé ou par étapes dans le temps d'un trouble du bilan en disulfure de thiol dans le cadre de la thérapie de substitution rénale ainsi que de tableaux morbides dans lesquels un trouble du bilan en disulfure de thiol de cellules immunitaires apparaît, où l'ambroxol de formule générale 1 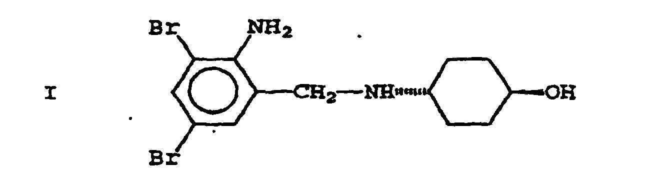 ses sels et/ou ses promédicaments est utilisé comme effecteur.

2. Médicament selon la revendication 1 **caractérisé en ce que** la dose d'acide α-lipoïque, de ses sels et/ou de ses promédicaments pour l'application en médecine humaine chez le patient est située entre 30 et 1200 mg/d, de préférence entre 200 et 600 mg/d.

3. Médicament selon la revendication 1 ou 2 **caractérisé en ce que** la dose d'ambroxol, de ses sels et/ou de ses promédicaments pour l'application en médecine humaine chez le patient est située entre 7,5 et 90 mg/d, de préférence entre 60 et 75 mg/d.

4. Médicament selon au moins l'une des revendications 1 à 3 **caractérisé en ce que** l'administration a lieu par voie orale ou parentérale.

5. Médicament selon au moins l'une des revendications 1 à 4 **caractérisé en ce que** d'autres additifs sont choisis dans le groupe des solvants aqueux, des stabilisants, des agents de suspension, de dispersion et de mouillage.

6. Médicament selon au moins l'une des revendications 1 à 5 sous forme d'une solution, de granulés, d'une poudre, d'une émulsion, d'un comprimé et/ou d'un comprimé pelliculé.

7. Utilisation d'au moins un effecteur du métabolisme du glutathion conjointement avec l'acide α-lipoïque, ses sels et/ou ses promédicaments pour la production d'un médicament pour le traitement simultané, séparé ou par étapes dans le temps d'un trouble du bilan en disulfure de thiol dans le cadre de la thérapie de substitution rénale sous forme d'un procédé d'hémodialyse ou de dialyse péritonéale.

8. Utilisation d'au moins un effecteur du métabolisme du glutathion conjointement avec l'acide α-lipoique, ses sels et/ou ses promédicaments pour la production d'un médicament pour le traitement simultané, séparé ou par étapes dans le temps d'un trouble du bilan en disulfure de thiol dans le cadre de tableaux morbides dans lesquels un trouble du bilan en disulfure de thiol de cellules immunitaires apparaît, en particulier pour le traitement immunomodulateur, augmentant les défenses et/ou anti-inflammatoire simultané, séparé ou par étapes dans le temps.

9. Utilisation selon les revendications 7 et 8 **caractérisée en ce qu'**un inhibiteur de l'enzyme de conversion de l'angiotensine (inhibiteur d'ACE) est utilisé comme effecteur.

10. Utilisation selon la revendication 9 **caractérisée en ce que** la dose de l'inhibiteur de l'enzyme de conversion de l'angiotensine (inhibiteur d'ACE) pour l'application en médecine humaine chez le patient est située entre 0,2 et 20 mg/d.

11. Utilisation selon la revendication 9 ou 10 **caractérisée en ce que** la 1-[(2S)-3-mercapto-2-méthylpropionyl]-L-proline (Captopril) de formule II 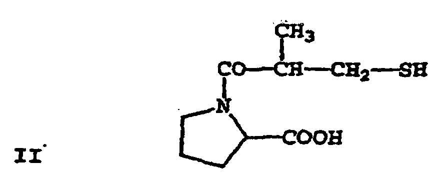 est utilisée comme inhibiteur d'ACE.

12. Utilisation selon la revendication 9 ou 10 **caractérisée en ce que** la 1-{N-[(S)-1-éthoxycarbonyl-3-phénylpropyl]-L-alanyl}-L-proline (Enalapril) de formule III 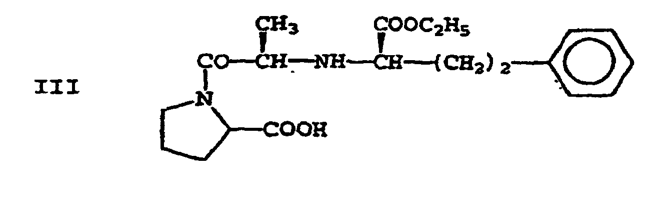 est utilisée comme inhibiteur d'ACE.

13. Utilisation selon la revendication 9 ou 10 **caractérisée en ce que** l'acide (2S,3aS,6aS)-1-{(S)-N-[(S)-1-éthoxycarbonyl-3-phénylpropyl]-alanyl}-octahydrocyclopenta[b]-pyrrole-2-carboxylique (Ramipril) de formule IV 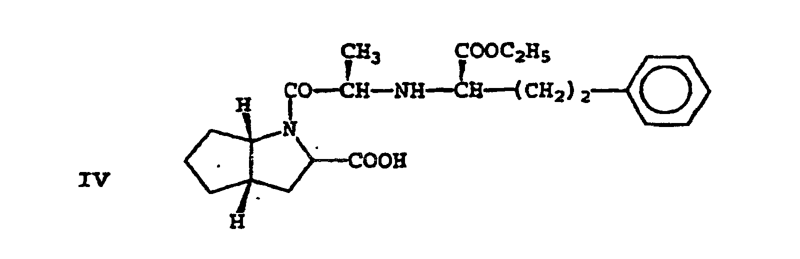 est utilisé comme inhibiteur d'ACE.

14. Utilisation selon au moins l'une des revendications 7 à 13 **caractérisée en ce que** l'effecteur du métabolisme du glutathion et l'acide α-lipoïque, ses sels et/ou ses promédicaments sont présents dans une formulation unique.

15. Utilisation selon au moins l'une des revendications 7 à 13 **caractérisée en ce que** l'effecteur du métabolisme du glutathion et l'acide α-lipoïque, ses sels et/ou ses promédicaments sont présents dans des formulations séparées.
